# FASCICULE DE BREVET EUROPEEN

(11) **EP 0 674 007 B1**
(45) Date de publication et mention de la délivrance du brevet: **21.07.1999**
(21) Numéro de dépôt: 94104402.6
(22) Date de dépôt: 21.03.1994
(51) Int. Cl.: C12P 17/16, C12N 5/04

(54) **Procédé de production de pilocarpine**
Verfahren zur Herstellung von Pilocarpin
Process for the preparation of pilocarpine

(43) Date de publication de la demande: 27.09.1995
(73) Titulaire: SOCIETE DES PRODUITS NESTLE S.A., 1800 Vevey (CH)
(72) Inventeur: Courtois, Didier, F-45000 Orleans (FR); Petiard, Vincent, F-37100 Tours (FR); Touche, André, F-37260 Monts (FR)
(74) Mandataire: Wavre, Claude-Alain

(56) Documents cités:
- EP-A- 0 283 051
- WO-A-90/12102
- US-A- 5 059 531

## Description

La présente invention a pour objet un procédé de production de pilocarpine.

La pilocarpine est un alcaloïde présentant des propriétés miotique et parasympathomimétique qui est à l'origine de diverses préparations pharmaceutiques et notamment ophtalmologiques. Utilisée comme miotique, elle permet par exemple de réduire la pression intraoculaire de l'oeil. La pilocarpine est ainsi employée de préférence dans le traitement du glaucome.

La production de pilocarpine est à ce jour principalement assurée par son extraction des feuilles de *Pilocarpus* sauvages. En effet, la production de pilocarpine par des méthodes chimiques ou biochimiques reste difficile et onéreuse. Cependant, la culture du *Pilocarpus* est longue, délicate et à ce jour peu développée. En effet, les graines de *Pilocarpus* ne conservent un pouvoir de germination que pendant un temps très court et les plantules poussent particulièrement lentement.

Le brevet US 5059531 décrit un procédé de multiplication végétative de plantes de *Pilocarpus*. Dans ce procédé, une suspension de cellules indifférenciées de *Pilocarpus* est soumise à un traitement hormonal qui induit la différenciation *in vitro* des cellules en bourgeons (ou feuilles). Ces bourgeons sont ensuite soumis à un traitement hormonal qui induit la formation de racines. On obtient ainsi des plantules qui seront par la suite cultivées en champs dans le but d'en récolter les feuilles. Ce procédé permet donc d'obtenir rapidement un grand nombre de plantules identiques de *Pilocarpus*, mais il ne résout pas les inconvénients liés à leur culture en champs.

Le brevet US ci-dessus décrit également la possibilité d'extraire directement la pilocarpine à partir des bourgeons induits. Cependant, cette culture *in vitro* de bourgeons présente des inconvénients. Premièrement, les bourgeons n'ont pas la capacité de se multiplier comme des cellules indifférenciées, la biomasse de la culture s'accroît ainsi uniquement à cause de l'allongement des bourgeons. La culture est donc longue et limitée en volume. Deuxièmement, la concentration de pilocarpine dans les bourgeons est inférieure d'un facteur dix à celle présente dans la plante, la quantité de pilocarpine que l'on peut purifier est donc également limitée.

La présente invention a pour but de pallier les inconvénients de l'art antérieur, et de proposer ainsi un procédé permettant la production aisée de pilocarpine.

A cet effet, on induit la formation de racines à partir d'une partie d'un végétal du genre *Pilocarpus*, on cultive *in vitro* lesdites racines dans un milieu de culture, et l'on isole la pilocarpine à partir des racines et/ou du milieu.

Dans un premier mode de réalisation de l'invention, on induit la formation de racines à partir d'un organe ou de cellules indifférenciées de *Pilocarpus* dans un milieu d'induction comprenant au moins une auxine et une cytokinine, on cultive *in vitro* lesdites racines dans un milieu de culture, puis on isole la pilocarpine à partir des racines et/ou du milieu.

De même, dans un deuxième mode de réalisation de l'invention, on induit la formation de racines à partir d'un organe de *Pilocarpus* à l'aide *d'Agrobacterium rhizogenes*, on cultive *in vitro* lesdites racines dans un milieu de culture, puis on isole la pilocarpine à partir des racines et/ou du milieu.

Dans la suite de la description, on emploiera l'expression "une partie d'un végétal" dans le sens de cellules indifférenciées ou organe de *Pilocarpus*.

On emploiera également l'expression "cellules indifférenciées" dans le sens de cellules qui ont une aptitude sous certaines conditions à se multiplier sous forme d'un cal ou d'une suspension cellulaire, puis sous certaines conditions à se différencier en un ou plusieurs types cellulaires qui peuvent s'organiser en un organe de plante, comme un bourgeon ou une racine, par exemple.

De même, on entend par le terme "cal" un amas macroscopique de cellules indifférenciées de plantes en culture sur un milieu de nutritif solide.

Enfin, on entend par l'expression "suspension cellulaire" des cellules indifférenciées pouvant former des amas microscopiques en culture dans un milieu de nutrition liquide.

La culture *in vitro* de racines de *Pilocarpus* en vue d'en isoler la pilocarpine présente des avantages réels par rapport aux méthodes traditionnelles. En effet, les racines de *Pilocarpus* ayant la capacité naturelle de s'allonger et de se multiplier, la culture de racine de *Pilocarpus* n'est pas limitée en volume et de plus est plus rapide que celle de bourgeons. En outre, la concentration en pilocarpine dans les racines étant équivalente à celle présente dans les feuilles de la plante d'origine, les racines constituent ainsi une source importante de pilocarpine. Enfin, le milieu de culture contient une quantité non négligeable de pilocarpine libérée par les racines, cette pilocarpine peut donc également être purifiée.

Pour mettre en oeuvre le premier mode de réalisation de l'invention, on induit donc la formation de racines à partir d'un organe ou de cellules indifférenciées de *Pilocarpus* dans un milieu d'induction comprenant au moins une auxine et une cytokinine.

Toutes les espèces de *Pilocarpus* peuvent servir de source d'organes ou de cellules pour la culture de racine selon la présente invention. On peut ainsi utiliser les espèces *Pilocarpus pennatifolius*, *Pilocarpus heterophyllus*, *Pilocarpus microphyllus* et *Pilocarpus jaborandi*, par exemple.

Dans le premier mode préférée de réalisation de l'invention, on peut donc induire une culture de racines à partir d'un organe de *Pilocarpus* à l'aide d'hormones végétales. Cet organe peut être un fragment de feuille, de racine, de tige ou de pièces florales issues de plantules ou d'arbres adultes. Pour cela, on peut stériliser l'organe qui a une longueur de 0.5 à 10 cm par des moyens connus, puis mettre l'organe sur un milieu d'induction solide contenant des hormones inductrices de la rhizogénèse à une température de 18°C à 34°C, de préférence à 24°C, et cultiver l'organe pendant quelques semaines selon un protocole standard à la lumière ou dans l'obscurité, de préférence dans l'obscurité. On peut obtenir ainsi une prolifération de racines à partir d'un ou plusieurs cals qui se sont formés sur l'organe dès les premières semaines de culture. Les dites racines présentent alors des structures racinaires identiques à celles des racines d'une plante.

Le milieu d'induction comprenant des hormones peut être un milieu de base habituellement utilisé en culture *in vitro* de cellules végétales. De préférence, on utilise le milieu de MURASHIGE et SKOOG complété des vitamines de SKOOG ou de LINSMAYER (Physiol. Plant., 1962, vol. 15, p. 473; Physiol. Plant., 1965, vol. 18, p. 100). On peut faire varier les concentrations des composants du milieu dans une certaine fourchette sans pour autant affecter le procédé selon la présente invention, en particulier on peut faire varier la concentration en glucides (saccharose, glucose) de 1 à 120 g/l, de préférence de 10 à 60 g/l. On peut également diminuer la teneur en macroéléments par un facteur 2. Enfin, la valeur du pH du milieu peut varier de 4 à 8, de préférence on utilise un pH d'environ 5,6 qui est le pH du milieu avant stérilisation.

De préférence, on utilise dans le milieu d'induction une auxine à une concentration de 0,1 à 10 mg/l, par exemple 1 mg/l, et une cytokinine à une concentration de 0,01 à 2 mg/l, par exemple 0,1 mg/l. De plus, l'auxine peut être choisie parmi les molécules suivantes: acide αnaphtalène acétique (ANA, acide βindol butyrique (AIB), acide 2,4-dichlorophénoxyacétique (2.4-D), acide βindol acétique (AIA), par exemple. Et la cytokinine peut être choisie parmi les molécules suivantes: kinétine (KIN), benzylaminopurine (BAP), isopentenyladénosine (IPA), par exemple.

La présence d'au moins une auxine et une cytokinine dans le milieu d'induction provoque ainsi au bout de quelques semaines, généralement 2 à 5, la formation d'un cal sur l'organe, et au bout de généralement six semaines la prolifération de racines. On peut également cultiver uniquement le cal. Pour cela, on met le cal obtenu dans les 5 premières semaines d'induction en culture sur un milieu nutritif semi-solide habituellement utilisé pour cultiver des cellules végétales *in vitro*. On peut ainsi conserver un cal pendant des années par repiquages successifs sur du milieu frais. On peut également repiquer le cal dans un milieu de culture traditionnel liquide agité. On obtient ainsi une suspension de cellules indifférenciées.

Dans le premier mode de réalisation de l'invention, on peut induire également la formation de racines à partir de cellules indifférenciées de *Pilocarpus* à l'aide d'hormones végétales. On peut soumettre ainsi ces cellules indifférenciées qui peuvent être un cal ou une suspension cellulaire, au traitement inductif de la rhizogénèse tel que décrit ci-dessus. On peut utiliser pour cela le même milieu de MURASHIGE et SKOOG et la même balance hormonale décrits pour le milieu d'induction précédent. De préférence, on utilise un milieu d'induction semi-solide pour un cal, et un milieu d'induction liquide pour une suspension cellulaire. On obtient ainsi une prolifération de racines à partir du cal ou des cellules indifférenciées en suspension.

Les racines obtenues selon le premier mode de réalisation de l'invention peuvent être alors cultivées *in vitro* sur un milieu de culture traditionnel, par exemple le milieu de MURASHIGE et SKOOG semi-solide ou liquide, de préférence liquide agité. Si les racines ont été obtenues à partir d'un cal ou d'un organe, on peut alors soit cultiver uniquement les racines c'est à dire séparer les racines du cal ou de l'organe, soit cultiver directement les racines avec le cal ou l'organe. En particulier, la balance hormonale du milieu de culture peut être différente de celle utilisée dans le milieu inductif de la rhizogénèse. On peut ainsi utiliser au moins une auxine à une concentration de 0.1 à 10 mg/l, ou utiliser en combinaison au moins une auxine à une concentration de 0.1 à 10 mg/l et une cytokinine à une concentration de 0.01 à 2 mg/l. On obtient ainsi une culture de racines dont la biomasse s'accroît par la multiplication et l'allongement desdites racines.

Dans un deuxième mode préférée de réalisation de l'invention, on induit la formation de racines à partir d'un organe de *Pilocarpus* à l'aide d'A*grobacterium rhizogenes*. En particulier pour induire la formation de racines, on peut incuber l'organe dans une suspension d'*Agrobacterium rhizogenes* de 30 minutes à 24 heures, puis on peut cultiver l'organe induit dans un milieu de nutrition jusqu'au développement de racines.

La suspension bactérienne peut être ainsi une culture traditionnelle d'*Agrobacterium rhizogenes* âgée d'au moins 20 heures, par exemple. De plus, après l'incubation on peut tuer les bactéries à l'aide d'un antibiotique.

En particulier, l'organe de *Pilocarpus* peut être un organe juvénile, c'est à dire un organe ou une partie d'organe de la plante qui est en cours de formation ou qui vient de se former, tels qu'un bourgeon, une jeune feuille, une jeune racine ou un embryon. De préférence, on choisit comme organe juvénile un bourgeon apical, ou un embryon zygotique, ou un organe différencié à partir d'un cal, par exemple un bourgeon ou un embryon somatique différencié grâce à un traitement traditionnel à l'aide d'hormones végétales.

Le milieu de nutrition peut être par ailleurs un milieu de base habituellement utilisé en culture *in vitro* de cellules végétales. De préférence, on utilise le milieu semi-solide de MURASHIGE et SKOOG complété des vitamines de SKOOG ou de LINSMAYER.

Certaines cellules de l'organe induit sont transformées par *Agrobacterium rhizogenes* et sont ainsi capables de produire naturellement des auxines. Par conséquent, on peut cultiver l'organe dans un milieu de nutrition dépourvu d'hormones végétales, sans pour autant diminuer notablement le nombre de pointes racinaires qui se forment. On peut cependant préférer ajouter au milieu de nutrition au moins une auxine afin de favoriser la survie de l'organe jusqu'au développement des racines. On peut ainsi utiliser une teneur en auxine habituellement utilisé pour cultiver *in vitro* un organe, par exemple. De même, on peut ajouter au milieu de nutrition au moins une auxine et une cytokinine à des teneurs telles qu'elles induisent un développement supplémentaire de racines, par exemple.

Des racines transformées de *Pilocarpus* vont alors se développer à partir de l'organe induit au bout d'environ 4 à 10 semaines. On obtient ainsi des racines du type "hairy root", que l'on peut repiquer sur un milieu de culture dépourvu d'hormones végétales. Ce milieu de culture peut être ainsi un milieu de base habituellement utilisé pour la culture *in vitro* de cellules végétales. De préférence, on utilise le milieu liquide de MURASHIGE et SKOOG complété des vitamines de SKOOG ou de LINSMAYER, par exemple. Enfin, on peut ajouter au milieu de culture au moins une auxine afin de favoriser la croissance des racines dans le milieu de culture, ou encore ajouter au moins une auxine et au moins une cytokinine à des teneurs telles qu'elles induisent un développement supplémentaire de racines, par exemple.

Finalement, dans le procédé selon la présente invention on extrait la pilocarpine à partir d'une culture de racines de *Pilocarpus* en utilisant les racines et/ou le milieu de culture. Cette culture peut avoir été obtenue selon l'un des deux modes d'induction de la rhizogénèse décrits ci-dessus. De préférence, on concentre, on sèche ou on lyophilise les racines et/ou le milieu de culture avant d'en extraire la pilocarpine.

Pour extraire, on peut utiliser une méthode de purification traditionnelle. On peut obtenir ainsi une quantité de pilocarpine par biomasse sèche équivalente à celle trouvée dans les feuilles de la plante d'origine. Par exemple, pour *Pilocarpus pennatifolius* on obtient de 300 à 500 µg/g de biomasse sèche, soit 300 à 500 mg de pilocarpine par kilogramme de biomasse.

La pilocarpine préparée selon la présente invention est identique à celle isolée à partir des feuilles par une méthode traditionnelle. On peut ainsi l'utiliser dans des spécialités pharmaceutiques appliquées notamment au traitement du glaucome.

La pilocarpine peut être purifiée et analysée plus en détail à l'aide des méthodes décrites ci-après. Les pourcentages sont donnés en volumes.

### Méthode de purification.

On extrait la pilocarpine biosynthétisée par une culture de racines de la manière suivante.

Les racines (ou cals rhizogènes) et/ou le milieu de culture sont séchés ou concentrés par une méthode traditionnelle. Les racines et/ou le milieu de culture sont broyés puis le tout est extrait sous agitation ou par percolation avec un mélange hydroalcoolique, de préférence l'alcool éthylique à 95° additionné d'un acide à des concentrations de 1 à 10%. On utilise de préférence l'acide chlorhydrique dans la proportion 99 (alcool): 1 (acide).

Le solide est filtré puis rincé avec le même solvant. Au filtrat est ajouté un volume d'eau, de préférence 30% (par rapport au volume d'alcool). L'alcool est évaporé sous vide (80 mbar, et à 50°C). La phase aqueuse concentrée est filtrée de nouveau pour éliminer l'insoluble aqueux.

Le filtrat est alors alcalinisé à des valeurs de pH comprises entre 5,5 et 9,5 avec des agents alcalins qui peuvent être des bases fortes diluées ou non ou des sels d'acides faibles et de bases fortes. Les alcaloïdes sont alors extraits à l'aide d'un solvant non miscible à l'eau, de préférence un solvant chloré, notamment le chloroforme ou le chlorure de méthylène. La phase aqueuse alcaline est extraite jusqu'à épuisement en alcaloïdes, par exemple en trois extractions successives.

Les phases organiques sont réunies, séchées sur un agent déshydratant, de préférence le sulfate de sodium. L'évaporation du solvant organique livre l'extrait alcaloïdique, dont l'alcaloïde majoritaire est la pilocarpine cristallisable sous forme de sels d'acide minéral ou organique.

### Analyse qualitative.

L'extrait alcaloïdique est analysé qualitativement en chromatographie couche mince sur gel de silice (Merck 60F254, Ref 5715) par élution mono ou bi-dimensionnelle avec comme éluants:
- Eluant 1: CHCl₃(90%)- MeOH (10%).
- Eluant 2: acétate d'éthyle (50%), méthyl éthylcétone (30%), acide formique (10%), eau (10%).

On révèle les alcaloïdes par le réactif de DRAGENDORFF.

### Analyse quantitative.

L'extrait alcaloïdique contenant la pilocarpine purifiée est analysé quantitativement en chromatographie liquide haute performance avec une colonne NOVAPAK C18 (Ref 86344, WATERS) de la manière suivante:
- Elution sous conditions isocratiques. Mélange tampon phosphate/acétonitrile avec des proportions de 1 à 5 de solvant organique.

On détecte les différents composés par absorption dans l'U.V. 220 nm ou par réfractomètrie. Les analyses sont effectuées par étalonnage externe avec le nitrate de pilocarpine.

L'extrait alcaloïdique contenant la pilocarpine purifiée peut être également analysé quantitativement en chromatographie en phase gazeuse avec une colonne capillaire CPSil 5CB CHROMPACK (Ref 7740). On utilise pour cela une température programmée de 150 à 220°C et on détecte par F.I.D. Les analyses sont effectuées par étalonnage externe avec le nitrate de pilocarpine ou par étalonnage interne avec le perchlorate de lupanine.

Les exemples suivants sont présentés à titre d'illustration du présent procédé de production de pilocarpine.

### Exemple 1

Obtention de racines *in vitro* à partir de fragments de feuilles de *Pilocarpus pennatifolius* Lem cultivés en serre.

Les feuilles sont prélevées sur des plants de *Pilocarpus pennatifolius* Lem. Après désinfection, le matériel végétal est découpé en fragments de 1 cm² déposés sur le milieu nutritif gélosé (semi-solide) MURASHIGE et SKOOG (complété des vitamines de SKOOG) contenant 1 mg/l d'ANA et 0,1 mg/l de KIN. Les cultures sont réalisées en boîtes de Pétri stériles (diamètre: 55 mm ou 100 mm), dans l'obscurité et à 24°C. Après 4 à 6 semaines de culture, les premières racines apparaissent à partir du cal qui s'est formé sur l'organe cultivé. Après au moins 6 semaines les racines obtenues sont séparées des fragments de feuilles ou de cals puis transférées sur le milieu semi-solide de MURASHIGE et SKOOG précédent et repiquées régulièrement sur ce même milieu.

### Exemple 2

Le matériel végétal décrit dans l'exemple 1 est déposé sur le milieu nutritif semi-solide MURASHIGE et SKOOG (complété des vitamines de LINSMAYER et dont la concentration en macroéléments est divisée par deux) contenant 1 mg/l d'ANA et 0,1 mg/l de BAP. Après 6 semaines les racines et le cal sont transférés directement sur le milieu précédent et repiqués régulièrement sur ce même milieu.

### Exemple 3

On utilise des cals de *Pilocarpus heterophyllus* repiqués depuis 6 années dans un milieu de culture standard.

On repique ces cals sur le milieu de MURASHIGE et SKOOG de base comprenant 10 mg/l d'ANA et 1 mg/l de KIN. Puis, on transfère ces cals sur le même milieu de base en présence de 1 mg/l d'ANA et 0,06 mg/l de KIN. On obtient un développement de racines au bout de quelques semaines.

### Exemple 4

Les racines obtenues dans l'exemple 1 sont repiquées sur le milieu liquide (sans gélose) de MURASHIGE et SKOOG complété des vitamines de LINSMAYER et comprenant 1 mg/l d'ANA et 0,1 mg/l de BAP.

Pour cela, on repique les racines toutes les 6 semaines pour un temps de doublement de 3 semaines et un taux d'inoculation de 20 gr de biomasse fraîche par litre de milieu. On cultive les racines en fioles d'Erlenmeyer de 250 ml (100 ml de milieu), à 24°C, dans l'obscurité et sous agitation à 100 rpm. Les racines se développent par allongement et ramification.

Les racines de l'exemple 2 (n'étant pas séparées de leur explant d'origine) sont également repiquées puis cultivées de la même manière que ci-dessus.

On obtient ainsi deux cultures de racines présentant sensiblement les mêmes caractéristiques de croissance.

### Exemple 5

Les cals de *Pilocarpus heterophyllus* tels que décrit dans l'exemple 3 sont transférés sur le milieu de base de MURASHIGE et SKOOG additionné de 2,4-D (0,5 mg/l) et de KIN (0,03 mg/l). Après 6 semaines, des embryons somatiques et des bourgeons se développent à partir des cals devenus organogènes. Ces cals sont ensuite incubés pendant 3 heures dans une suspension bactérienne d'A*grobacterium rhizogenes* âgée de 24 heures. La souche bactérienne utilisée est une souche A4 virulente contenant le plasmide Ri sauvage et le plasmide binaire p35S-GUS-INT (G. Vancanneyt et al., Mol. Gen. Genet.,1990, 245-250) et cultivée sur le milieu MYA (M. TEPFER et al., Microbiol. Sci., 1987, 24-28).

Le plasmide binaire p35S-GUS-INT comprend le gène rapporteur GUS et le gène de sélection NPTII. La présence de l'intron PIV2 dans la phase codante du gène GUS permet d'éviter une éventuelle expression du gène GUS due à des bactéries résiduelles.

Après incubation, les cals organogènes sont transférés sur le milieu de nutrition de MURASHIGE et SKOOG additionné de 2,4-D (0,5 mg/l) et de KIN (0,03 mg/l). Au bout de 2 semaines, des échantillons de matériel végétal sont soumis à un test histochimique d'activité de la β-glucoronidase selon le protocole de R.A. Jefferson (Plant Molecular Biology Reporter, Vol. 5, n°4, 1987, 387-405). La coloration bleue observée caractérise l'expression du gène GUS. Des analyses par PCR et southern blot traditionnelles (J. Spiral et al., in ASIC, 15ième colloque, Montpellier, 1993) montrent par ailleurs son intégration dans le génome de cellules végétales.

Au bout d'environ 4 semaines, des racines se développent à partir des organes différencié du cal. On transfère alors ces organes et ces racines dans le milieu de culture liquide de MURASHIGE et SKOOG dépourvu d'hormones végétales. On observe alors un développement continu de racines transformées.

### Exemple 6

Les cals organogènes incubés dans une suspension d'*Agrobacterium rhizogenes* tels que décrits l'exemple 5, sont transférés sur le milieu de nutrition de MURASHIGE et SKOOG additionné de 0,5 mg/l de 2,4-D. Au bout d'environ 5 semaines, des racines se développent à partir des organes. On transfère alors uniquement les racines dans le milieu de culture liquide de MURASHIGE et SKOOG comprenant 1 mg/l d'ANA. On observe alors un développement continu de racines transformées.

### Exemple 7

Les cals organogènes incubés dans une suspension d'*Agrobacterium rhizogenes* tels que décrits l'exemple 5, sont transférés sur le milieu de nutrition de MURASHIGE et SKOOG dépourvu d'hormones végétales. Au bout d'environ 5 semaines, des racines se développent à partir des organes. On transfère alors uniquement les racines dans le milieu de culture liquide de MURASHIGE et SKOOG comprenant 1 mg/l d'ANA et 0,1 mg/l de KIN. On observe alors un développement continu de racines transformées.

### Exemple 8

La culture de racines telle que décrite dans l'exemple 1 est placée en bioréacteur (NBS, France) de 10 litres à agitation par pales, à une densité d'inoculation de 20 g par litre. Les conditions de culture et la croissance des racines sont identiques à celles observées en fioles d'Erlenmeyer (agitation: 100 rpm, aération: 0,5 v/v/min).

### Exemple 9

La pilocarpine peut être extraite des cultures de racines telles que décrites dans les exemples 1 à 8, par la méthode de purification décrite ci-dessus. Les quantités de pilocarpine obtenues à partir de ces cultures et déterminées par la méthode quantitative décrite ci-dessus, sont de l'ordre de 300 à 500 µg/g de matière sèche. Ces quantités sont équivalentes à celles trouvées dans les feuilles de la plante d'origine.

## Revendications

1. Procédé de production de pilocarpine, dans lequel on induit la formation de racines à partir d'une partie d'un végétal du genre *Pilocarpus*, on cultive *in vitro* lesdites racines dans un milieu de culture, et l'on isole la pilocarpine à partir des racines et/ou du milieu.

2. Procédé selon la revendication 1, dans lequel on induit la formation de racines à partir d'un organe ou de cellules indifférenciées de *Pilocarpus* dans un milieu d'induction comprenant au moins une auxine et une cytokinine, on cultive *in vitro* lesdites racines dans un milieu de culture, et l'on isole la pilocarpine à partir des racines et/ou du milieu.

3. Procédé selon la revendication 2, caractérisé en ce que le milieu d'induction comprend une auxine à une concentration de 0,1 à 10 mg/l et une cytokinine à une concentration de 0,01 à 2 mg/l.

4. Procédé selon la revendication 2, caractérisé en ce que le milieu de culture comprend au moins une auxine à une concentration de 0,1 à 10 mg/l ou une combinaison d'au moins une auxine à une concentration de 0,1 à 10 mg/l et une cytokinine à une concentration de 0,01 à 2 mg/l.

5. Procédé selon la revendication 2, caractérisé en ce que l'organe est un fragment de feuille, de racine, de tige ou de pièces florales issues de plantules ou d'arbres adultes, et que les cellules indifférenciées sont un cal ou une suspension cellulaire.

6. Procédé selon la revendication 1, dans lequel on induit la formation de racines à partir d'un organe de *Pilocarpus* à l'aide d'*Agrobacterium rhizogenes*, on cultive *in vitro* lesdites racines dans un milieu de culture, puis on isole la pilocarpine à partir des racines et/ou du milieu.

7. Procédé selon la revendication 6, caractérisé par le fait que pour induire la formation de racines, on incube premièrement l'organe dans une suspension d'*Agrobacterium rhizogenes* pendant 30 minutes à 24 heures, puis on cultive l'organe induit dans un milieu de nutrition jusqu'au développement de racines.

8. Procédé selon la revendication 7, caractérisé par le fait que soit le milieu de nutrition est dépourvu d'hormones végétales, soit il comprend au moins une auxine, soit il comprend en combinaison au moins une auxine et une cytokinine.

9. Procédé selon la revendication 6, caractérisé par le fait que soit le milieu de culture est dépourvu d'hormones végétales, soit il comprend au moins une auxine, soit il comprend en combinaison au moins une auxine et une cytokinine.

10. Procédé selon la revendication 6, caractérisé en ce que l'organe de *Pilocarpus* est un organe juvénile.

11. Procédé selon la revendication 10, caractérisé en ce que l'organe juvénile est choisi dans le groupe formé par un bourgeon apical, un embryon somatique, et un organe différencié à partir d'un cal.

12. Procédé selon l'une des revendications 2, 6 et 7, caractérisé en ce que les milieux d'induction et/ou de nutrition et/ou de culture sont chacun le milieu MURASHIGE et SKOOG complété des vitamines de SKOOG ou de LINSMAYER.

13. Procédé selon la revendication 1, caractérisé en ce que les racines sont cultivées dans un milieu de culture liquide.

14. Procédé selon la revendication 1, caractérisé en ce que les racines de *Pilocarpus* sont des racines de *Pilocarpus pennatifolius*, ou de *Pilocarpus heterophyllus*, ou de *Pilocarpus microphyllus* ou de *Pilocarpus jaborandi*.

## Patentansprüche

1. Verfahren zur Herstellung von Pilocarpin, bei dem man die Bildung von Wurzeln ausgehend von einem Teil einer Pflanze der Gattung *Pilocarpus* induziert, diese Wurzeln *in vitro* in einem Kulturmedium kultiviert und Pilocarpin aus den Wurzeln und/oder dem Medium isoliert.

2. Verfahren nach Anspruch 1, bei dem man die Bildung von Wurzeln ausgehend von einem Organ oder undifferenzierten Zellen von *Pilocarpus* in einem Induktionsmedium induziert, das wenigstens ein Auxin und ein Cytokinin enthält, die Wurzeln *in vitro* in einem Kulturmedium kultiviert und Pilocarpin aus den Wurzeln und/oder dem Medium isoliert.

3. Verfahren nach Anspruch 2, dadurch gekennzeichnet, daß das Induktionsmedium ein Auxin in einer Konzentration von 0,1 bis 10 mg/l und ein Cytokinin in einer Konzentration von 0,1 bis 2 mg/l enthält.

4. Verfahren nach Anspruch 2, dadurch gekennzeichnet, daß das Kulturmedium wenigstens ein Auxin in einer Konzentration von 0,1 bis 10 mg/l oder eine Kombination von wenigstens einem Auxin in einer Konzentration von 0,1 bis 10 mg/l und einem Cytokinin in einer Konzentration von 0,01 bis 2 mg/l enthält.

5. Verfahren nach Anspruch 2, dadurch gekennzeichnet, daß das Organ ein Fragment eines Blatts, einer Wurzel, eines Sprosses oder eines Blütenteils ist, die aus Pflänzchen oder erwachsenen Bäumen stammen, und daß die undifferenzierten Zellen ein Kallus oder eine Zellsuspension sind.

6. Verfahren nach Anspruch 1, bei dem man die Bildung von Wurzeln ausgehend von einem Organ von *Pilocarpus* mit Hilfe von *Agrobacterium rhizogenes* induziert, die Wurzeln *in vitro* in einem Kulturmedium kultiviert, und danach Pilocarpin aus den Wurzeln und/oder dem Medium isoliert.

7. Verfahren nach Anspruch 6, dadurch gekennzeichnet, daß man zur Induzierung der Wurzelbildung das Organ zuerst in einer Suspension von *Agrobacterium rhizogenes* für 30 min bis 24 h inkubiert, danach das induzierte Organ in einem Nährmedium kultiviert, bis sich Wurzeln entwickeln.

8. Verfahren nach Anspruch 7, dadurch gekennzeichnet, daß entweder das Nährmedium arm an Pflanzenhormonen ist, oder daß es wenigstens ein Auxin enthält, oder daß es in Kombination wenigstens ein Auxin und ein Cytokinin enthält.

9. Verfahren nach Anspruch 6, dadurch gekennzeichnet, daß das Kulturmedium entweder arm an Pflanzenhormonen ist, oder daß es wenigstens ein Auxin enthält, oder daß es in Kombination wenigstens ein Auxin und ein Cytokinin enthält.

10. Verfahren nach Anspruch 6, dadurch gekennzeichnet, daß das Organ von *Pilocarpus* ein juveniles Organ ist.

11. Verfahren nach Anspruch 10, dadurch gekennzeichnet, daß das juvenile Organ aus der Gruppe ausgewählt ist, die gebildet wird von einer apikalen Knospe, einem somatischen Embryo und einem differenzierten Organ aus einem Kallus.

12. Verfahren nach einem der Ansprüche 2, 6 und 7, dadurch gekennzeichnet, daß das Induktions- und/oder das Nähr- und/oder das Kulturmedium jeweils das Medium von MURASHIGE und SKOOG, ergänzt durch die SKOOG- oder LINSMAYER-Vitamine, ist.

13. Verfahren nach Anspruch 1, dadurch gekennzeichnet, daß die Wurzeln in einem flüssigen Kulturmedium kultiviert werden.

14. Verfahren nach Anspruch 1, dadurch gekennzeichnet, daß die *Pilocarpus-Wurzeln* die Wurzeln von *Pilocarpus pennatifolius* oder von *Pilocarpus heterophyllus* oder von *Pilocarpus microphyllus* oder von *Pilocarpus jaborandi,* sind.

## Claims

1. Process for the production of pilocarpine, in which root formation is induced from part of a plant of the genus *Pilocarpus,* the said roots are cultivated *in vitro* in a culture medium and pilocarpine is isolated from the roots and/or the medium.

2. Process according to Claim 1, in which root formation is induced from an organ or undifferentiated cells of *Pilocarpus* in an induction medium comprising at least one auxine and one cytokinine, the said roots are cultivated *in vitro* in a culture medium and pilocarpine is isolated from the roots and/or the medium.

3. Process according to claim 2, characterized in that the induction medium comprises an auxine at a concentration of 0.1 to 10 mg/l and a cytokinine at a concentration of 0.01 to 2 mg/l.

4. Process according to claim 2, characterized in that the culture medium comprises at least one auxine at a concentration of 0.1 to 10 mg/l or a combination of at least one auxine at a concentration of 0.1 to 10 mg/l and one cytokinine at a concentration of 0.01 to 2 mg/l.

5. Process according to Claim 2, characterized in that the organ is a fragment of leaf, root, or stem or parts of flowers from plantlets or adult trees, and that the undifferentiated cells are a callus or a cell suspension.

6. Process according to Claim 1, in which root formation is induced from an organ of *Pilocarpus* with the aid of in vitro *Agrobacterium rhizogenes,* the said roots are cultivated in a culture medium and pilocarpine is then isolated from the roots and/or medium.

7. Process according to Claim 6, characterized in that, in order to induce root formation, the organ is first incubated in a suspension of *Agrobacterium rhizogenes* for 30 minutes to 24 hours, and the induced organ is then cultivated in a nutrient medium until roots develop.

8. Process according to Claim 7, characterized in that either the nutrient medium does not contain plant hormones, or it comprises at least one auxine, or it comprises a combination of at least one auxine and one cytokinine.

9. Process according to Claim 6, characterized in that either the culture medium does not contain plant hormones, or it comprises at least one auxine, or it comprises a combination of at least one auxine and one cytokinine.

10. Process according to Claim 6, characterized in that the *Pilocarpus* organ is a juvenile organ.

11. Process according to Claim 10, characterized in that the juvenile organ is selected from the group formed of an apical bud, a somatic embryo and an organ differentiated from a callus.

12. Process according to one of Claims 2, 6 and 7, characterized in that the induction and/or nutrient and/or culture media are each the MURASHIGE and SKOOG medium with the addition of SKOOG or LINSMAYER vitamins.

13. Process according to Claim 1, characterized in that roots are cultivated in a liquid culture medium.

14. Process according to Claim 1, characterized in that the roots of *Pilocarpus* are roots of *Pilocarpus pennatifolius,* or of *Pilocarpus heterophyllus,* or of *Pilocarpus microphyllus,* or of *Pilocarpus jaborandi.*
